# EUROPEAN PATENT APPLICATION

(11) **EP 4 014 966 A1**
(43) Date of publication of application: **22.06.2022**
(21) Application number: 21208072.5
(22) Date of filing: 06.07.2012
(51) Int. Cl.: A61K 9/127, A61K 39/39, A61K 39/155, A61K 39/00, A61P 37/04, A61K 39/12

(54) **LIPOSOMES HAVING USEFUL N:P RATIO FOR DELIVERY OF RNA MOLECULES**

(30) Priority: 06.07.2011 US 201161505088 P
(62) Divisional of application: 20214948.0
(71) Applicant: GlaxoSmithKline Biologicals S.A., 1330 Rixensart (BE)
(72) Inventor: GEALL, Andrew, Emeryville, CA 94608-2916 (US); VERMA, Ayush, Emeryville, CA 94608-2916 (US)
(74) Representative: West, Heloise

(57) **Abstract**

Nucleic acid immunisation is achieved by delivering a RNA encapsulated within a liposome comprising a cationic lipid, wherein the liposome and the RNA have a N:P ratio of between 1:1 and 20:1.

## Description

This application claims the benefit of U.S. Provisional Application No. 61/505,088, which was filed July 6, 2011, the complete contents of which are hereby incorporated herein by reference for all purposes.

### TECHNICAL FIELD

This invention is in the field of non-viral delivery of RNAs for immunisation.

### BACKGROUND ART

The delivery of nucleic acids for immunising animals has been a goal for several years. Various approaches have been tested, including the use of DNA or RNA, of viral or non-viral delivery vehicles (or even no delivery vehicle, in a "naked" vaccine), of replicating or non-replicating vectors, or of viral or non-viral vectors.

There remains a need for further and improved nucleic acid vaccines and, in particular, for improved ways of delivering nucleic acid vaccines.

### DISCLOSURE OF THE INVENTION

According to the invention, nucleic acid immunisation is achieved by delivering a RNA encapsulated within a liposome comprising a cationic lipid, wherein the liposome and the RNA have a N:P ratio of between 1:1 and 20:1. The "N:P ratio" refers to the molar ratio of nitrogen atoms in the cationic lipid to phosphates in the RNA. The liposomes are useful for *in vivo* delivery of RNA to a vertebrate cell.

Thus the invention provides a liposome in which a RNA is encapsulated, wherein the liposome comprises a cationic lipid, the RNA encodes an immunogen, and the liposome & RNA have a N:P ratio of between 1:1 and 20:1.

The invention also provides a process for preparing a liposome which encapsulates an immunogen-encoding RNA, wherein the liposome is formed by mixing liposome-forming components with an immunogen-encoding RNA, wherein the liposome-forming ingredients comprise a cationic lipid, and wherein the cationic lipid and the RNA are mixed at a N:P ratio of between 1:1 and 20:1.

### The liposome

Various amphiphilic lipids can form bilayers in an aqueous environment to encapsulate a RNA-containing aqueous core as a liposome. These lipids can have an anionic, cationic or zwitterionic hydrophilic head group. Formation of liposomes from anionic phospholipids dates back to the 1960s, and cationic liposome-forming lipids have been studied since the 1990s. Some phospholipids are anionic whereas other are zwitterionic and others are cationic. Suitable classes of phospholipid include, but are not limited to, phosphatidylethanolamines, phosphatidylcholines, phosphatidylserines, and phosphatidyl-glycerols, and some useful phospholipids are listed in Table 1. Useful cationic lipids include, but are not limited to, dioleoyl trimethylammonium propane (DOTAP), 1,2-distearyloxy-N,N-dimethyl-3-aminopropane (DSDMA), 1,2-dioleyloxy-N,Ndimethyl-3-aminopropane (DODMA), 1,2-dilinoleyloxy-N,N-dimethyl-3-aminopropane (DLinDMA), 1,2-dilinolenyloxy-N,N-dimethyl-3-aminopropane (DLenDMA). Zwitterionic lipids include, but are not limited to, acyl zwitterionic lipids and ether zwitterionic lipids. Examples of useful zwitterionic lipids are DPPC, DOPC and dodecylphosphocholine. The lipids can be saturated or unsaturated. The use of at least one unsaturated lipid for preparing liposomes is preferred. If an unsaturated lipid has two tails, both tails can be unsaturated, or it can have one saturated tail and one unsaturated tail.

Liposomal particles of the invention can be formed from a single lipid or from a mixture of lipids, provided that at least one cationic lipid is used. Where a mixture is used, it may comprise both saturated and unsaturated lipids. For example, a mixture useful with the invention may comprise DlinDMA (cationic, unsaturated), DSPC (zwitterionic, saturated), and/or DMG (anionic, saturated). Where a mixture of lipids is used, not all of the component lipids in the mixture need to be charged *e.g.* one or more amphiphilic lipids, including a cationic lipid, can be mixed with cholesterol.

Liposomal particles of the invention include at least one cationic lipid whose head group includes at least one nitrogen atom which is capable of being protonated. The number of these lipid nitrogen atoms which can be protonated in the liposome contribute to the "N:P ratio" used herein.

Preferred cationic lipids have a protonatable nitrogen with a pKa in the range of 5.0 to 7.6. Ideally the lipid with a pKa in this range has a tertiary amine; such lipids behave differently from lipids such as DOTAP or DC-Chol, which have a quaternary amine group. At physiological pH amines with a pKa in the range of 5.0 to 7.6 have neutral or reduced surface charge, whereas a lipid such as DOTAP is strongly cationic. The inventors have found that liposomes formed from quaternary amine lipids (e.g. DOTAP) are less suitable for delivery of immunogen-encoding RNA than liposomes formed from tertiary amine lipids (e.g. DLinDMA).

Within this pKa range, preferred lipids have a pKa of 5.5 to 6.7 *e.g.* between 5.6 and 6.8, between 5.6 and 6.3, between 5.6 and 6.0, between 5.5 and 6.2, or between 5.7 and 5.9. The pKa is the pH at which 50% of the lipids are charged, lying halfway between the point where the lipids are completely charged and the point where the lipids are completely uncharged. It can be measured in various ways, but is preferably measured using the method disclosed below in the section entitled "pKa measurement". The pKa typically should be measured for the lipid alone rather than for the lipid in the context of a mixture which also includes other lipids (e.g. not as performed in reference 5, which looks at the pKa of a SNALP rather than of the individual lipids).

Preferred lipids with a pKa in this range have a tertiary amine. For example, they may comprise 1,2-dilinoleyloxy-N,N-dimethyl-3-aminopropane (DLinDMA; pKa 5.8) and/or 1,2-dilinolenyloxy-N,N-dimethyl-3-aminopropane (DLenDMA). Another suitable lipid having a tertiary amine is 1,2-dioleyloxy-N,Ndimethyl-3-aminopropane (DODMA). See ref. 1. Some of the amino acid lipids of reference 2 may also be used, as can certain of the amino lipids of reference 3. Further useful lipids with tertiary amines in their headgroups are disclosed in reference 4, the complete contents of which are incorporated herein by reference.

The hydrophilic portion of a lipid can be PEGylated (*i.e.* modified by covalent attachment of a polyethylene glycol). This modification can increase stability and prevent non-specific adsorption of the liposomes. For instance, lipids can be conjugated to PEG using techniques such as those disclosed in reference 1 and 5. Various lengths of PEG can be used e.g. between 0.5-8kDa.

Where a liposome is formed from a mixture of lipids, it is preferred that the proportion of those lipids which have a protonatable nitrogen should be between 20-80% of the total amount of lipids e.g. between 30-70%, or between 40-60%. The remainder of the lipid content can be made of *e.g.* cholesterol *(e.g.* 35-50% cholesterol) and/or DMG (optionally PEGylated) and/or DSPC. Such mixtures are used below. These % values are mole percentages. A mixture of DSPC, DlinDMA, PEG-DMG and cholesterol is used in the examples.

Liposomal particles are usually divided into three groups: multilamellar vesicles (MLV); small unilamellar vesicles (SUV); and large unilamellar vesicles (LUV). MLVs have multiple bilayers in each vesicle, forming several separate aqueous compartments. SUVs and LUVs have a single bilayer encapsulating an aqueous core; SUVs typically have a diameter ≤50nm, and LUVs have a diameter >50nm. Liposomal particles of the invention are ideally LUVs with a diameter in the range of 50-220nm. For a composition comprising a population of LUVs with different diameters: (i) at least 80% by number should have diameters in the range of 20-220nm, (ii) the average diameter (Zav, by intensity) of the population is ideally in the range of 40-200nm, and/or (iii) the diameters should have a polydispersity index <0.2.

The lipid DLinDMA is sometimes referred to herein as RV01; this is a preferred cationic lipid for use with the invention, although in some embodiments the liposomes does not comprise DLinDMA:

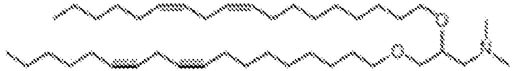

Another useful lipid, disclosed in reference 4, is sometimes referred to herein as RV05:

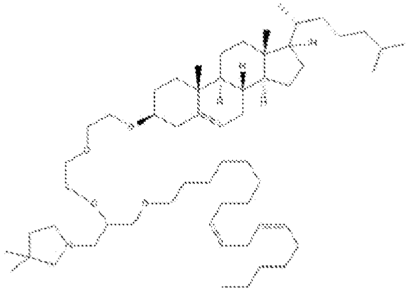

The lipid DOTAP is sometimes referred to herein as RV13, but it is not a preferred lipid.

### Mixing process

Techniques for preparing suitable liposomes are well known in the art *e.g.* see references 6 to 8. One useful method is described in reference 9 and involves mixing (i) an ethanolic solution of the lipids (ii) an aqueous solution of the nucleic acid and (iii) buffer, followed by mixing, equilibration, dilution and purification. Preferred liposomes of the invention are obtainable by this mixing process.

A useful process for preparing a RNA-containing liposome comprises steps of: (a) mixing RNA with a lipid at a pH which is below the lipid's pKa but is above 4.5; then (b) increasing the pH to be above the lipid's pKa. Thus a cationic lipid is positively charged during liposome formation in step (a), but the pH change thereafter means that the majority (or all) of the positively charged groups become neutral. This process is advantageous for preparing liposomes of the invention, and by avoiding a pH below 4.5 during step (a) the stability of the encapsulated RNA is improved.

The pH in step (a) is above 4.5, and is ideally above 4.8. Using a pH in the range of 5.0 to 6.0, or in the range of 5.0 to 5.5, can provide suitable liposomes.

The increased pH in step (b) is above the lipid's pKa. The pH is ideally increased to a pH less than 9, and preferably less than 8. Depending on the lipid's pKa, the pH in step (b) may thus be increased to be within the range of 6 to 8 *e.g.* to pH 6.5±0.3. The pH increase of step (b) can be achieved by transferring the liposomes into a suitable buffer e.g. into phosphate-buffered saline. The pH increase of step (b) is ideally performed after liposome formation has taken place.

RNA used in step (a) can be in aqueous solution, for mixing with an organic solution of the lipid *(e.g.* an ethanolic solution, as in ref. 9). The mixture can then be diluted to form liposomes, after which the pH can be increased in step (b).

### The RNA

Liposomes of the invention include a RNA molecule which (unlike siRNA) encodes an immunogen. After *in vivo* administration of the liposomes, RNA is released and is translated inside a cell to provide the immunogen *in situ.*

The RNA is +-stranded, and so it can be translated without needing any intervening replication steps such as reverse transcription. Preferred +-stranded RNAs are self-replicating, unlike reference 10. A self-replicating RNA molecule (replicon) can, when delivered to a cell even without any proteins, lead to the production of multiple daughter RNAs by transcription from itself (via an antisense copy which it generates from itself). A self-replicating RNA molecule is thus typically a +-strand molecule which can be directly translated after delivery to a cell, and this translation provides a RNA-dependent RNA polymerase which then produces both antisense and sense transcripts from the delivered RNA. Thus the delivered RNA leads to the production of multiple daughter RNAs. These daughter RNAs, as well as collinear subgenomic transcripts, may be translated themselves to provide *in situ* expression of an encoded immunogen, or may be transcribed to provide further transcripts with the same sense as the delivered RNA which are translated to provide *in situ* expression of the immunogen. The overall results of this sequence of transcriptions is a huge amplification in the number of the introduced replicon RNAs and so the encoded immunogen becomes a major polypeptide product of the cells.

One suitable system for achieving self-replication in this manner is to use an alphavirus-based replicon. These replicons are +-stranded RNAs which lead to translation of a replicase (or replicase-transcriptase) after delivery to a cell. The replicase is translated as a polyprotein which auto-cleaves to provide a replication complex which creates genomic --strand copies of the +-strand delivered RNA. These --strand transcripts can themselves be transcribed to give further copies of the +-stranded parent RNA and also to give a subgenomic transcript which encodes the immunogen. Translation of the subgenomic transcript thus leads to *in situ* expression of the immunogen by the infected cell. Suitable alphavirus replicons can use a replicase from a Sindbis virus, a Semliki forest virus, an eastern equine encephalitis virus, a Venezuelan equine encephalitis virus, *etc.* Mutant or wild-type virus sequences can be used *e.g.* the attenuated TC83 mutant of VEEV has been used in replicons [11].

A preferred self-replicating RNA molecule thus encodes (i) a RNA-dependent RNA polymerase which can transcribe RNA from the self-replicating RNA molecule and (ii) an immunogen. The polymerase can be an alphavirus replicase e.g. comprising one or more of alphavirus proteins nsP1, nsP2, nsP3 and nsP4.

Whereas natural alphavirus genomes encode structural virion proteins in addition to the non-structural replicase polyprotein, it is preferred that the self-replicating RNA molecules of the invention do not encode alphavirus structural proteins. Thus a preferred self-replicating RNA can lead to the production of genomic RNA copies of itself in a cell, but not to the production of RNA-containing virions. The inability to produce these virions means that, unlike a wild-type alphavirus, the self-replicating RNA molecule cannot perpetuate itself in infectious form. The alphavirus structural proteins which are necessary for perpetuation in wild-type viruses are absent from self-replicating RNAs of the invention and their place is taken by gene(s) encoding the immunogen of interest, such that the subgenomic transcript encodes the immunogen rather than the structural alphavirus virion proteins.

Thus a self-replicating RNA molecule useful with the invention may have two open reading frames. The first (5') open reading frame encodes a replicase; the second (3') open reading frame encodes an immunogen. In some embodiments the RNA may have additional (e.g. downstream) open reading frames e.g. to encode further immunogens (see below) or to encode accessory polypeptides.

A preferred self-replicating RNA molecule has a 5' cap *(e.g.* a 7-methylguanosine). This cap can enhance *in vivo* translation of the RNA. In some embodiments the 5' sequence of the self-replicating RNA molecule must be selected to ensure compatibility with the encoded replicase.

A self-replicating RNA molecule may have a 3' poly-A tail. It may also include a poly-A polymerase recognition sequence *(e.g.* AAUAAA) near its 3' end.

Self-replicating RNA molecules can have various lengths but they are typically 5000-25000 nucleotides long *e.g.* 8000-15000 nucleotides, or 9000-12000 nucleotides. Thus the RNA is longer than seen in siRNA delivery.

Self-replicating RNA molecules will typically be single-stranded. Single-stranded RNAs can generally initiate an adjuvant effect by binding to TLR7, TLR8, RNA helicases and/or PKR. RNA delivered in double-stranded form (dsRNA) can bind to TLR3, and this receptor can also be triggered by dsRNA which is formed either during replication of a single-stranded RNA or within the secondary structure of a single-stranded RNA.

The self-replicating RNA can conveniently be prepared by *in vitro* transcription (IVT). IVT can use a (cDNA) template created and propagated in plasmid form in bacteria, or created synthetically (for example by gene synthesis and/or polymerase chain-reaction (PCR) engineering methods). For instance, a DNA-dependent RNA polymerase (such as the bacteriophage T7, T3 or SP6 RNA polymerases) can be used to transcribe the self-replicating RNA from a DNA template. Appropriate capping and poly-A addition reactions can be used as required (although the replicon's poly-A is usually encoded within the DNA template). These RNA polymerases can have stringent requirements for the transcribed 5' nucleotide(s) and in some embodiments these requirements must be matched with the requirements of the encoded replicase, to ensure that the IVT-transcribed RNA can function efficiently as a substrate for its self-encoded replicase.

As discussed in reference 12, the self-replicating RNA can include (in addition to any 5' cap structure) one or more nucleotides having a modified nucleobase. For instance, a self-replicating RNA can include one or more modified pyrimidine nucleobases, such as pseudouridine and/or 5-methylcytosine residues. In some embodiments, however, the RNA includes no modified nucleobases, and may include no modified nucleotides *i.e.* all of the nucleotides in the RNA are standard A, C, G and U ribonucleotides (except for any 5' cap structure, which may include a 7'-methylguanosine). In other embodiments, the RNA may include a 5' cap comprising a 7'-methylguanosine, and the first 1, 2 or 3 5' ribonucleotides may be methylated at the 2' position of the ribose.

A RNA used with the invention ideally includes only phosphodiester linkages between nucleosides, but in some embodiments it can contain phosphoramidate, phosphorothioate, and/or methylphosphonate linkages.

Ideally, a liposome includes fewer than 10 different species of RNA *e.g.* 5, 4, 3, or 2 different species; most preferably, a liposome includes a single RNA species *i.e.* all RNA molecules in the liposome have the same sequence and same length.

### The N:P ratio

The invention uses liposomes prepared to have a N:P ratio of between 1:1 and 20:1. As mentioned above, the "N:P ratio" refers to the molar ratio of protonatable nitrogen atoms in the liposome's cationic lipids (typically solely in the lipid's headgroup) to phosphates in the RNA.

Useful N:P ratios are from about 2:1 to about 18:1, from about 4:1 to 16:1, from about 6:1 to about 14:1, from about 8:1 to about 12:1. Preferred N:P ratios are between 3:1 and 11:1. Useful N:P ratios as exemplified below include 2:1, 4:1, 8:1 and 10:1.

As shown below, the N:P ratio can have an impact on immunogenicity, with lower ratios being preferred *e.g.* lower than 8:1, lower than 7:1, lower than 6:1, lower than 5:1, or ≤4:1 *e.g.* between 2:1 and 4:1 inclusive.

In some embodiments the N:P ratio is not between 7:1 and 9:1. In some embodiments the N:P ratio is not between 7.5:1 and 8.5:1. In some embodiments the N:P ratio is not 8:1.

The N:P ratio can be modified by varying the proportions of cationic lipid and RNA during liposome formation. This is achieved most conveniently by modifying the RNA concentration in aqueous material which is used during formation of liposomes from a suitable combination of liposome-forming components. For example, in the method mentioned above the ethanolic solution of liposome-forming lipids can be kept constant while the concentration of RNA in the aqueous solution is varied. A higher RNA concentration will decrease the N:P ratio, whereas a lower RNA concentration will increase the N:P ratio.

For certain purposes the N:P ratio can be based on assumptions. For instance, if µg of a RNA molecule is assumed to contain 3 nmol of anionic phosphate, and each µg of DlinDMA is assumed to contains 1.6 nmol of cationic nitrogen, the calculation can be simplified for convenience.

### The immunogen

RNA molecules used with the invention encode a polypeptide immunogen. After administration of the liposomes the immunogen is translated *in vivo* and can elicit an immune response in the recipient. The immunogen may elicit an immune response against a bacterium, a virus, a fungus or a parasite (or, in some embodiments, against an allergen; and in other embodiments, against a tumor antigen). The immune response may comprise an antibody response (usually including IgG) and/or a cell-mediated immune response. The polypeptide immunogen will typically elicit an immune response which recognises the corresponding bacterial, viral, fungal or parasite (or allergen or tumour) polypeptide, but in some embodiments the polypeptide may act as a mimotope to elicit an immune response which recognises a bacterial, viral, fungal or parasite saccharide. The immunogen will typically be a surface polypeptide e.g. an adhesin, a hemagglutinin, an envelope glycoprotein, a spike glycoprotein, *etc.*

RNA molecules can encode a single polypeptide immunogen or multiple polypeptides. Multiple immunogens can be presented as a single polypeptide immunogen (fusion polypeptide) or as separate polypeptides. If immunogens are expressed as separate polypeptides then one or more of these may be provided with an upstream IRES or an additional viral promoter element. Alternatively, multiple immunogens may be expressed from a polyprotein that encodes individual immunogens fused to a short autocatalytic protease (e.g. foot-and-mouth disease virus 2A protein), or as inteins.

The RNA encodes an immunogen. For the avoidance of doubt, the invention does not encompass RNA which encodes a firefly luciferase or which encodes a fusion protein of *E.coli* β-galactosidase or which encodes a green fluorescent protein (GFP). Also, the RNA is not total mouse thymus RNA. Also, the invention does not encompass RNA which encodes a secreted alkaline phosphatase.

In some embodiments the immunogen elicits an immune response against one of these bacteria:
*Neisseria meningitidis:* useful immunogens include, but are not limited to, membrane proteins such as adhesins, autotransporters, toxins, iron acquisition proteins, and factor H binding protein. A combination of three useful polypeptides is disclosed in reference 13.
*Streptococcus pneumoniae:* useful polypeptide immunogens are disclosed in reference 14. These include, but are not limited to, the RrgB pilus subunit, the beta-N-acetyl-hexosaminidase precursor (spr0057), spr0096, General stress protein GSP-781 (spr2021, SP2216), serine/threonine kinase StkP (SP1732), and pneumococcal surface adhesin PsaA.
*Streptococcus pyogenes:* useful immunogens include, but are not limited to, the polypeptides disclosed in references 15 and 16.
*Moraxella catarrhalis.*
*Bordetella pertussis:* Useful pertussis immunogens include, but are not limited to, pertussis toxin or toxoid (PT), filamentous haemagglutinin (FHA), pertactin, and agglutinogens 2 and 3.
*Staphylococcus aureus:* Useful immunogens include, but are not limited to, the polypeptides disclosed in reference 17, such as a hemolysin, esxA, esxB, ferrichrome-binding protein (sta006) and/or the sta011 lipoprotein.
*Clostridium tetani:* the typical immunogen is tetanus toxoid.
*Cornynebacterium diphtheriae:* the typical immunogen is diphtheria toxoid.
*Haemophilus influenzae:* Useful immunogens include, but are not limited to, the polypeptides disclosed in references 18 and 19.
*Pseudomonas aeruginosa*
*Streptococcus agalactiae:* useful immunogens include, but are not limited to, the polypeptides disclosed in reference 15.
*Chlamydia trachomatis:* Useful immunogens include, but are not limited to, PepA, LcrE, ArtJ, DnaK, CT398, OmpH-like, L7/L12, OmcA, AtoS, CT547, Eno, HtrA and MurG (*e.g*. as disclosed in reference 20. LcrE [21] and HtrA [22] are two preferred immunogens.
*Chlamydia pneumoniae:* Useful immunogens include, but are not limited to, the polypeptides disclosed in reference 23.
*Helicobacter pylori:* Useful immunogens include, but are not limited to, CagA, VacA, NAP, and/or urease [24].
*Escherichia coli:* Useful immunogens include, but are not limited to, immunogens derived from enterotoxigenic *E. coli* (ETEC), enteroaggregative *E. coli* (EAggEC), diffusely adhering *E*. *coli* (DAEC), enteropathogenic *E. coli* (EPEC), extraintestinal pathogenic *E. coli* (ExPEC) and/or enterohemorrhagic *E. coli* (EHEC). ExPEC strains include uropathogenic *E.coli* (UPEC) and meningitis/sepsis-associated *E.coli* (MNEC). Useful UPEC polypeptide immunogens are disclosed in references 25 and 26. Useful MNEC immunogens are disclosed in reference 27. A useful immunogen for several *E.coli* types is AcfD [28].
*Bacillus anthracis*
*Yersinia pestis:* Useful immunogens include, but are not limited to, those disclosed in references 29 and 30.
*Staphylococcus epidermis*
*Clostridium perfringens* or *Clostridium botulinums*
*Legionella pneumophila*
*Coxiella burnetii*
*Brucella,* such as *B.abortus, B.canis, B.melitensis, B.neotomae, B.ovis, B.suis, B.pinnipediae.*
*Francisella,* such as *F.novicida, F.philomiragia, F.tularensis.*
*Neisseria gonorrhoeae*
*Treponema pallidum*
*Haemophilus ducreyi*
*Enterococcus faecalis* or *Enterococcus faecium*
*Staphylococcus saprophyticus*
*Yersinia enterocolitica*
*Mycobacterium tuberculosis*
*Rickettsia*
*Listeria monocytogenes*
*Vibrio cholerae*
*Salmonella typhi*
*Borrelia burgdorferi*
*Porphyromonas gingivalis*
*Klebsiella*

In some embodiments the immunogen elicits an immune response against one of these viruses:
*Orthomyxovirus:* Useful immunogens can be from an influenza A, B or C virus, such as the hemagglutinin, neuraminidase or matrix M2 proteins. Where the immunogen is an influenza A virus hemagglutinin it may be from any subtype e.g. H1, H2, H3, H4, H5, H6, H7, H8, H9, H10, H11, H12, H13, H14, H15 or H16.
*Paramyxoviridae* viruses: Viral immunogens include, but are not limited to, those derived from Pneumoviruses *(e.g.* respiratory syncytial virus, RSV), Rubulaviruses *(e.g.* mumps virus), Paramyxoviruses *(e.g.* parainfluenza virus), Metapneumoviruses and Morbilliviruses *(e.g.* measles). In some embodiments, however, the immunogen is not a RSV protein.
*Poxviridae:* Viral immunogens include, but are not limited to, those derived from *Orthopoxvirus* such as *Variola vera,* including but not limited to, *Variola major* and *Variola minor.*
*Picornavirus:* Viral immunogens include, but are not limited to, those derived from Picornaviruses, such as Enteroviruses, Rhinoviruses, Heparnavirus, Cardioviruses and Aphthoviruses. In one embodiment, the enterovirus is a poliovirus e.g. a type 1, type 2 and/or type 3 poliovirus. In another embodiment, the enterovirus is an EV71 enterovirus. In another embodiment, the enterovirus is a coxsackie A or B virus.
*Bunyavirus:* Viral immunogens include, but are not limited to, those derived from an *Orthobunyavirus,* such as California encephalitis virus, a *Phlebovirus,* such as Rift Valley Fever virus, or a *Nairovirus,* such as *Crimean-Congo hemorrhagic fever* virus.
*Heparnavirus:* Viral immunogens include, but are not limited to, those derived from a Heparnavirus, such as hepatitis A virus (HAV).
*Filovirus:* Viral immunogens include, but are not limited to, those derived from a filovirus, such as an Ebola virus (including a Zaire, Ivory Coast, Reston or Sudan ebolavirus) or a Marburg virus.
*Togavirus:* Viral immunogens include, but are not limited to, those derived from a Togavirus, such as a Rubivirus, an Alphavirus, or an Arterivirus. This includes rubella virus.
*Flavivirus:* Viral immunogens include, but are not limited to, those derived from a Flavivirus, such as Tick-borne encephalitis (TBE) virus, Dengue (types 1, 2, 3 or 4) virus, Yellow Fever virus, Japanese encephalitis virus, Kyasanur Forest Virus, West Nile encephalitis virus, St. Louis encephalitis virus, Russian spring-summer encephalitis virus, Powassan encephalitis virus.
*Pestivirus:* Viral immunogens include, but are not limited to, those derived from a Pestivirus, such as Bovine viral diarrhea (BVDV), Classical swine fever (CSFV) or Border disease (BDV).
*Hepadnavirus:* Viral immunogens include, but are not limited to, those derived from a Hepadnavirus, such as Hepatitis B virus. A composition can include hepatitis B virus surface antigen (HBsAg).
*Other hepatitis viruses:* A composition can include an immunogen from a hepatitis C virus, delta hepatitis virus, hepatitis E virus, or hepatitis G virus.
*Rhabdovirus:* Viral immunogens include, but are not limited to, those derived from a Rhabdovirus, such as a Lyssavirus (e.g. a Rabies virus) and Vesiculovirus (VSV).
*Caliciviridae:* Viral immunogens include, but are not limited to, those derived from Calciviridae, such as Norwalk virus (Norovirus), and Norwalk-like Viruses, such as Hawaii Virus and Snow Mountain Virus.
*Coronavirus:* Viral immunogens include, but are not limited to, those derived from a SARS coronavirus, avian infectious bronchitis (IBV), Mouse hepatitis virus (MHV), and Porcine transmissible gastroenteritis virus (TGEV). The coronavirus immunogen may be a spike polypeptide.
*Retrovirus:* Viral immunogens include, but are not limited to, those derived from an Oncovirus, a Lentivirus (e.g. HIV-1 or HIV-2) or a Spumavirus.
*Reovirus:* Viral immunogens include, but are not limited to, those derived from an Orthoreovirus, a Rotavirus, an Orbivirus, or a Coltivirus.
*Parvovirus:* Viral immunogens include, but are not limited to, those derived from Parvovirus B19.
*Herpesvirus:* Viral immunogens include, but are not limited to, those derived from a human herpesvirus, such as, by way of example only, Herpes Simplex Viruses (HSV) (e.g. HSV types 1 and 2), Varicella-zoster virus (VZV), Epstein-Barr virus (EBV), Cytomegalovirus (CMV), Human Herpesvirus 6 (HHV6), Human Herpesvirus 7 (HHV7), and Human Herpesvirus 8 (HHV8).
*Papovaviruses:* Viral immunogens include, but are not limited to, those derived from Papillomaviruses and Polyomaviruses. The (human) papillomavirus may be of serotype 1, 2, 4, 5, 6, 8, 11, 13, 16, 18, 31, 33, 35, 39, 41, 42, 47, 51, 57, 58, 63 or 65 *e.g.* from one or more of serotypes 6, 11, 16 and/or 18.
*Adenovirus:* Viral immunogens include those derived from adenovirus serotype 36 (Ad-36).

In some embodiments, the immunogen elicits an immune response against a virus which infects fish, such as: infectious salmon anemia virus (ISAV), salmon pancreatic disease virus (SPDV), infectious pancreatic necrosis virus (IPNV), channel catfish virus (CCV), fish lymphocystis disease virus (FLDV), infectious hematopoietic necrosis virus (IHNV), koi herpesvirus, salmon picorna-like virus (also known as picorna-like virus of atlantic salmon), landlocked salmon virus (LSV), atlantic salmon rotavirus (ASR), trout strawberry disease virus (TSD), coho salmon tumor virus (CSTV), or viral hemorrhagic septicemia virus (VHSV).

Fungal immunogens may be derived from Dermatophytres, including: *Epidermophyton floccusum, Microsporum audouini, Microsporum canis, Microsporum distortum, Microsporum equinum, Microsporum gypsum, Microsporum nanum, Trichophyton concentricum, Trichophyton equinum, Trichophyton gallinae, Trichophyton gypseum, Trichophyton megnini, Trichophyton mentagrophytes, Trichophyton quinckeanum, Trichophyton rubrum, Trichophyton schoenleini, Trichophyton tonsurans, Trichophyton verrucosum, T. verrucosum* var. album, var. discoides, var. ochraceum, *Trichophyton violaceum,* and/or *Trichophyton faviforme;* or from *Aspergillus fumigatus, Aspergillus flavus, Aspergillus niger, Aspergillus nidulans, Aspergillus terreus, Aspergillus sydowi, Aspergillus flavatus, Aspergillus glaucus, Blastoschizomyces capitatus, Candida albicans, Candida enolase, Candida tropicalis, Candida glabrata, Candida krusei, Candida parapsilosis, Candida stellatoidea, Candida kusei, Candida parakwsei, Candida lusitaniae, Candida pseudotropicalis, Candida guilliermondi, Cladosporium carrionii, Coccidioides immitis, Blastomyces dermatidis, Cryptococcus neoformans, Geotrichum clavatum, Histoplasma capsulatum, Klebsiella pneumoniae, Microsporidia, Encephalitozoon* spp., *Septata intestinalis* and *Enterocytozoon bieneusi;* the less common are *Brachiola* spp, *Microsporidium* spp., *Nosema* spp., *Pleistophora* spp., *Trachipleistophora* spp., *Vittaforma* spp *Paracoccidioides brasiliensis, Pneumocystis carinii, Pythiumn insidiosum, Pityrosporum ovale, Sacharomyces cerevisae, Saccharomyces boulardii, Saccharomyces pombe, Scedosporium apiosperum, Sporothrix schenckii, Trichosporon beigelii, Toxoplasma gondii, Penicillium marneffei, Malassezia spp., Fonsecaea spp., Wangiella spp., Sporothrix spp., Basidiobolus spp., Conidiobolus spp., Rhizopus spp, Mucor spp, Absidia spp, Mortierella spp, Cunninghamella spp, Saksenaea spp., Alternaria spp, Curvularia spp, Helminthosporium spp, Fusarium spp, Aspergillus spp, Penicillium spp, Monolinia spp, Rhizoctonia spp, Paecilomyces spp, Pithomyces spp,* and *Cladosporium spp.*

In some embodiments the immunogen elicits an immune response against a parasite from the *Plasmodium* genus, such as *P.falciparum, P.vivax, P.malariae* or *P.ovale.* Thus the invention may be used for immunising against malaria. In some embodiments the immunogen elicits an immune response against a parasite from the *Caligidae* family, particularly those from the *Lepeophtheirus* and *Caligus* genera *e.g.* sea lice such as *Lepeophtheirus salmonis* or *Caligus rogercresseyi.*

In some embodiments the immunogen elicits an immune response against: pollen allergens (tree-, herb, weed-, and grass pollen allergens); insect or arachnid allergens (inhalant, saliva and venom allergens, e.g. mite allergens, cockroach and midges allergens, hymenopthera venom allergens); animal hair and dandruff allergens (from *e.g.* dog, cat, horse, rat, mouse, *etc.*); and food allergens *(e.g.* a gliadin). Important pollen allergens from trees, grasses and herbs are such originating from the taxonomic orders of Fagales, Oleales, Pinales and platanaceae including, but not limited to, birch (Betula), alder (Alnus), hazel (Corylus), hornbeam (Carpinus) and olive (Olea), cedar (Cryptomeria and Juniperus), plane tree (Platanus), the order of Poales including grasses of the genera Lolium, Phleum, Poa, Cynodon, Dactylis, Holcus, Phalaris, Secale, and Sorghum, the orders of Asterales and Urticales including herbs of the genera Ambrosia, Artemisia, and Parietaria. Other important inhalation allergens are those from house dust mites of the genus Dermatophagoides and Euroglyphus, storage mite e.g. Lepidoglyphys, Glycyphagus and Tyrophagus, those from cockroaches, midges and fleas *e.g.* Blatella, Periplaneta, Chironomus and Ctenocepphalides, and those from mammals such as cat, dog and horse, venom allergens including such originating from stinging or biting insects such as those from the taxonomic order of Hymenoptera including bees (*Apidae*)*,* wasps (*Vespidea*), and ants (*Formicoidae*)*.*

In some embodiments the immunogen is a tumor antigen selected from: (a) cancer-testis antigens such as NY-ESO-1, SSX2, SCP1 as well as RAGE, BAGE, GAGE and MAGE family polypeptides, for example, GAGE-1, GAGE-2, MAGE-1, MAGE-2, MAGE-3, MAGE-4, MAGE-5, MAGE-6, and MAGE-12 (which can be used, for example, to address melanoma, lung, head and neck, NSCLC, breast, gastrointestinal, and bladder tumors; (b) mutated antigens, for example, p53 (associated with various solid tumors, *e.g.,* colorectal, lung, head and neck cancer), p21/Ras (associated with, *e.g.,* melanoma, pancreatic cancer and colorectal cancer), CDK4 (associated with, *e.g*., melanoma), MUM1 (associated with, *e.g.,* melanoma), caspase-8 (associated with, *e.g.,* head and neck cancer), CIA 0205 (associated with, *e.g.,* bladder cancer), HLA-A2-R1701, beta catenin (associated with, *e.g.,* melanoma), TCR (associated with, *e.g*., T-cell non-Hodgkins lymphoma), BCR-abl (associated with, *e.g*., chronic myelogenous leukemia), triosephosphate isomerase, KIA 0205, CDC-27, and LDLR-FUT; (c) over-expressed antigens, for example, Galectin 4 (associated with, *e.g*., colorectal cancer), Galectin 9 (associated with, *e.g.,* Hodgkin's disease), proteinase 3 (associated with, *e.g.,* chronic myelogenous leukemia), WT 1 (associated with, *e.g*., various leukemias), carbonic anhydrase (associated with, *e.g.,* renal cancer), aldolase A (associated with, *e.g.,* lung cancer), PRAME (associated with, *e.g.,* melanoma), HER-2/neu (associated with, *e.g.,* breast, colon, lung and ovarian cancer), mammaglobin, alpha-fetoprotein (associated with, *e.g.*, hepatoma), KSA (associated with, *e.g.,* colorectal cancer), gastrin (associated with, *e.g.,* pancreatic and gastric cancer), telomerase catalytic protein, MUC-1 (associated with, *e.g*., breast and ovarian cancer), G-250 (associated with, *e.g.,* renal cell carcinoma), p53 (associated with, *e.g.,* breast, colon cancer), and carcinoembryonic antigen (associated with, *e.g*., breast cancer, lung cancer, and cancers of the gastrointestinal tract such as colorectal cancer); (d) shared antigens, for example, melanoma-melanocyte differentiation antigens such as MART-1/Melan A, gp100, MC1R, melanocyte-stimulating hormone receptor, tyrosinase, tyrosinase related protein-1/TRP1 and tyrosinase related protein-2/TRP2 (associated with, *e.g*., melanoma); (e) prostate associated antigens such as PAP, PSA, PSMA, PSH-P1, PSM-P1, PSM-P2, associated with *e.g.*, prostate cancer; (f) immunoglobulin idiotypes (associated with myeloma and B cell lymphomas, for example). In certain embodiments, tumor immunogens include, but are not limited to, p15, Hom/Mel-40, H-Ras, E2A-PRL, H4-RET, IGH-IGK, MYL-RAR, Epstein Barr virus antigens, EBNA, human papillomavirus (HPV) antigens, including E6 and E7, hepatitis B and C virus antigens, human T-cell lymphotropic virus antigens, TSP-180, p185erbB2, p180erbB-3, c-met, mn-23H1, TAG-72-4, CA 19-9, CA 72-4, CAM 17.1, NuMa, K-ras, p16, TAGE, PSCA, CT7, 43-9F, 5T4, 791 Tgp72, beta-HCG, BCA225, BTAA, CA 125, CA 15-3 (CA 27.29\BCAA), CA 195, CA 242, CA-50, CAM43, CD68\KP1, CO-029, FGF-5, Ga733 (EpCAM), HTgp-175, M344, MA-50, MG7-Ag, MOV18, NB/70K, NY-CO-1, RCAS1, SDCCAG16, TA-90 (Mac-2 binding protein/cyclophilin C-associated protein), TAAL6, TAG72, TLP, TPS, and the like.

### Pharmaceutical compositions

Liposomes of the invention are useful as components in pharmaceutical compositions for immunising subjects against various diseases. These compositions will typically include a pharmaceutically acceptable carrier in addition to the liposomes. A thorough discussion of pharmaceutically acceptable carriers is available in reference 31.

A pharmaceutical composition of the invention may include one or more small molecule immunopotentiators. For example, the composition may include a TLR2 agonist (e.g. Pam3CSK4), a TLR4 agonist *(e.g.* an aminoalkyl glucosaminide phosphate, such as E6020), a TLR7 agonist *(e.g.* imiquimod), a TLR8 agonist *(e.g.* resiquimod) and/or a TLR9 agonist *(e.g.* IC31). Any such agonist ideally has a molecular weight of <2000Da. In some embodiments such agonist(s) are also encapsulated in the liposome (e.g. together with the RNA), but in other embodiments they are unencapsulated.

Pharmaceutical compositions of the invention may include the particles in plain water (e.g. w.f.i.) or in a buffer *e.g.* a phosphate buffer, a Tris buffer, a borate buffer, a succinate buffer, a histidine buffer, or a citrate buffer. Buffer salts will typically be included in the 5-20mM range.

Pharmaceutical compositions of the invention may have a pH between 5.0 and 9.5 *e.g.* between 6.0 and 8.0.

Compositions of the invention may include sodium salts (e.g. sodium chloride) to give tonicity. A concentration of 10±2 mg/ml NaCl is typical e.g. about 9 mg/ml.

Compositions of the invention may include metal ion chelators. These can prolong RNA stability by removing ions which can accelerate phosphodiester hydrolysis. Thus a composition may include one or more of EDTA, EGTA, BAPTA, pentetic acid, *etc..* Such chelators are typically present at between 10-500µM *e.g.* 0.1mM. A citrate salt, such as sodium citrate, can also act as a chelator, while advantageously also providing buffering activity.

Pharmaceutical compositions of the invention may have an osmolality of between 200 mOsm/kg and 400 mOsm/kg, e.g. between 240-360 mOsm/kg, or between 290-310 mOsm/kg.

Pharmaceutical compositions of the invention may include one or more preservatives, such as thiomersal or 2-phenoxyethanol. Mercury-free compositions are preferred, and preservative-free vaccines can be prepared.

Pharmaceutical compositions of the invention are preferably sterile.

Pharmaceutical compositions of the invention are preferably non-pyrogenic e.g. containing <1 EU (endotoxin unit, a standard measure) per dose, and preferably <0.1 EU per dose.

Pharmaceutical compositions of the invention are preferably gluten free.

Pharmaceutical compositions of the invention may be prepared in unit dose form. In some embodiments a unit dose may have a volume of between 0.1-1.0ml e.g. about 0.5ml.

The compositions may be prepared as injectables, either as solutions or suspensions. The composition may be prepared for pulmonary administration e.g. by an inhaler, using a fine spray. The composition may be prepared for nasal, aural or ocular administration e.g. as spray or drops. Injectables for intramuscular administration are typical.

Compositions comprise an immunologically effective amount of particles, as well as any other components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (e.g. non-human primate, primate, *etc.*)*,* the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. The particle and RNA content of compositions of the invention will generally be expressed in terms of the amount of RNA per dose. A preferred dose has ≤100µg RNA (e.g. from 10-100µg, such as about 10µg, 25µg, 50µg, 75µg or 100µg), but expression can be seen even at lower levels.

The invention also provides a delivery device (e.g. syringe, nebuliser, sprayer, inhaler, dermal patch, *etc.*) containing a pharmaceutical composition of the invention. This device can be used to administer the composition to a vertebrate subject.

Particles of the invention do not include ribosomes.

### Methods of treatment and medical uses

Liposomes and pharmaceutical compositions of the invention are for *in vivo* use for eliciting an immune response against an immunogen of interest.

The invention provides a method for raising an immune response in a vertebrate comprising the step of administering an effective amount of a liposome or pharmaceutical composition of the invention. The immune response is preferably protective and preferably involves antibodies and/or cell-mediated immunity. The method may raise a booster response.

The invention also provides a liposome or pharmaceutical composition of the invention for use in a method for raising an immune response in a vertebrate.

The invention also provides the use of a liposome of the invention in the manufacture of a medicament for raising an immune response in a vertebrate.

By raising an immune response in the vertebrate by these uses and methods, the vertebrate can be protected against various diseases and/or infections e.g. against bacterial and/or viral diseases as discussed above. The particles and compositions are immunogenic, and are more preferably vaccine compositions. Vaccines according to the invention may either be prophylactic (*i.e.* to prevent infection) or therapeutic (*i.e.* to treat infection), but will typically be prophylactic.

The vertebrate is preferably a mammal, such as a human or a veterinary mammal (e.g. small animals, such as dogs or cats; or large animals, such as horses, cattle, deer, goats, pigs); in some embodiments, the vertebrate is not a mouse or a cotton rat or a cow. Where the vaccine is for prophylactic use, the human is preferably a child *(e.g.* a toddler or infant) or a teenager; where the vaccine is for therapeutic use, the human is preferably a teenager or an adult. A vaccine intended for children may also be administered to adults *e.g.* to assess safety, dosage, immunogenicity, *etc.*

Vaccines prepared according to the invention may be used to treat both children and adults. Thus a human patient may be less than 1 year old, less than 5 years old, 1-5 years old, 5-15 years old, 15-55 years old, or at least 55 years old. Preferred patients for receiving the vaccines are the elderly *(e.g.* ≥50 years old, ≥60 years old, and preferably ≥65 years), the young (e.g. ≤5 years old), hospitalised patients, healthcare workers, armed service and military personnel, pregnant women, the chronically ill, or immunodeficient patients. The vaccines are not suitable solely for these groups, however, and may be used more generally in a population.

Compositions of the invention will generally be administered directly to a patient. Direct delivery may be accomplished by parenteral injection (e.g. subcutaneously, intraperitoneally, intravenously, intramuscularly, intradermally, or to the interstitial space of a tissue). Alternative delivery routes include rectal, oral *(e.g.* tablet, spray), buccal, sublingual, vaginal, topical, transdermal or transcutaneous, intranasal, ocular, aural, pulmonary or other mucosal administration. Intradermal and intramuscular administration are two preferred routes. Injection may be via a needle *(e.g.* a hypodermic needle), but needle-free injection may alternatively be used. A typical intramuscular dose is 0.5 ml.

The invention may be used to elicit systemic and/or mucosal immunity, preferably to elicit an enhanced systemic and/or mucosal immunity.

Dosage can be by a single dose schedule or a multiple dose schedule. Multiple doses may be used in a primary immunisation schedule and/or in a booster immunisation schedule. In a multiple dose schedule the various doses may be given by the same or different routes *e.g.* a parenteral prime and mucosal boost, a mucosal prime and parenteral boost, *etc.* Multiple doses will typically be administered at least 1 week apart (e.g. about 2 weeks, about 3 weeks, about 4 weeks, about 6 weeks, about 8 weeks, about 10 weeks, about 12 weeks, about 16 weeks, *etc.*)*.* In one embodiment, multiple doses may be administered approximately 6 weeks, 10 weeks and 14 weeks after birth, e.g. at an age of 6 weeks, 10 weeks and 14 weeks, as often used in the World Health Organisation's Expanded Program on Immunisation ("EPI"). In an alternative embodiment, two primary doses are administered about two months apart, e.g. about 7, 8 or 9 weeks apart, followed by one or more booster doses about 6 months to 1 year after the second primary dose, e.g. about 6, 8, 10 or 12 months after the second primary dose. In a further embodiment, three primary doses are administered about two months apart, e.g. about 7, 8 or 9 weeks apart, followed by one or more booster doses about 6 months to 1 year after the third primary dose, e.g. about 6, 8, 10, or 12 months after the third primary dose.

### General

The practice of the present invention will employ, unless otherwise indicated, conventional methods of chemistry, biochemistry, molecular biology, immunology and pharmacology, within the skill of the art. Such techniques are explained fully in the literature. See, *e.g.,* references 32-38, *etc.*

The term "comprising" encompasses "including" as well as "consisting" e.g. a composition "comprising" X may consist exclusively of X or may include something additional e.g. X + Y.

The term "about" in relation to a numerical value x is optional and means, for example, *x*±10%.

The word "substantially" does not exclude "completely" *e.g.* a composition which is "substantially free" from Y may be completely free from Y. Where necessary, the word "substantially" may be omitted from the definition of the invention.

References to charge, to cations, to anions, to zwitterions, *etc.,* are taken at pH 7.

TLR3 is the Toll-like receptor 3. It is a single membrane-spanning receptor which plays a key role in the innate immune system. Known TLR3 agonists include poly(I:C). "TLR3" is the approved HGNC name for the gene encoding this receptor, and its unique HGNC ID is HGNC: 11849. The RefSeq sequence for the human TLR3 gene is GI:2459625.

TLR7 is the Toll-like receptor 7. It is a single membrane-spanning receptor which plays a key role in the innate immune system. Known TLR7 agonists include e.g. imiquimod. "TLR7" is the approved HGNC name for the gene encoding this receptor, and its unique HGNC ID is HGNC:15631. The RefSeq sequence for the human TLR7 gene is GI:67944638.

TLR8 is the Toll-like receptor 8. It is a single membrane-spanning receptor which plays a key role in the innate immune system. Known TLR8 agonists include e.g. resiquimod. "TLR8" is the approved HGNC name for the gene encoding this receptor, and its unique HGNC ID is HGNC:15632. The RefSeq sequence for the human TLR8 gene is GI:20302165.

The RIG-I-like receptor ("RLR") family includes various RNA helicases which play key roles in the innate immune system[39]. RLR-1 (also known as RIG-I or retinoic acid inducible gene I) has two caspase recruitment domains near its N-terminus. The approved HGNC name for the gene encoding the RLR-1 helicase is "DDX58" (for DEAD (Asp-Glu-Ala-Asp) box polypeptide 58) and the unique HGNC ID is HGNC:19102. The RefSeq sequence for the human RLR-1 gene is GI:77732514. RLR-2 (also known as MDA5 or melanoma differentiation-associated gene 5) also has two caspase recruitment domains near its N-terminus. The approved HGNC name for the gene encoding the RLR-2 helicase is "IFIH1" (for interferon induced with helicase C domain 1) and the unique HGNC ID is HGNC:18873. The RefSeq sequence for the human RLR-2 gene is GI: 27886567. RLR-3 (also known as LGP2 or laboratory of genetics and physiology 2) has no caspase recruitment domains. The approved HGNC name for the gene encoding the RLR-3 helicase is "DHX58" (for DEXH (Asp-Glu-X-His) box polypeptide 58) and the unique HGNC ID is HGNC:29517. The RefSeq sequence for the human RLR-3 gene is GI:149408121.

PKR is a double-stranded RNA-dependent protein kinase. It plays a key role in the innate immune system. "EIF2AK2" (for eukaryotic translation initiation factor 2-alpha kinase 2) is the approved HGNC name for the gene encoding this enzyme, and its unique HGNC ID is HGNC:9437. The RefSeq sequence for the human PKR gene is GI:208431825.

### MODES FOR CARRYING OUT THE INVENTION

### RNA replicons

Various replicons are used below. In general these are based on a hybrid alphavirus genome with non-structural proteins from venezuelan equine encephalitis virus (VEEV), a packaging signal from sindbis virus, and a 3' UTR from Sindbis virus or a VEEV mutant. The replicon is about 10kb long and has a poly-A tail.

Plasmid DNA encoding alphavirus replicons (named: pT7-mVEEV-FL.RSVF or A317; pT7-mVEEV-SEAP or A306; pSP6-VCR-GFP or A50) served as a template for synthesis of RNA *in vitro.* The replicons contain the alphavirus genetic elements required for RNA replication but lack those encoding gene products necessary for particle assembly; the structural proteins are instead replaced by a protein of interest (either a reporter, such as SEAP or GFP, or an immunogen, such as full-length RSV F protein) and so the replicons are incapable of inducing the generation of infectious particles. A bacteriophage (T7 or SP6) promoter upstream of the alphavirus cDNA facilitates the synthesis of the replicon RNA *in vitro* and a hepatitis delta virus (HDV) ribozyme immediately downstream of the poly(A)-tail generates the correct 3'-end through its self-cleaving activity.

Following linearization of the plasmid DNA downstream of the HDV ribozyme with a suitable restriction endonuclease, run-off transcripts were synthesized in vitro using T7 or SP6 bacteriophage derived DNA-dependent RNA polymerase. Transcriptions were performed for 2 hours at 37°C in the presence of 7.5 mM (T7 RNA polymerase) or 5 mM (SP6 RNA polymerase) of each of the nucleoside triphosphates (ATP, CTP, GTP and UTP) following the instructions provided by the manufacturer (Ambion). Following transcription the template DNA was digested with TURBO DNase (Ambion). The replicon RNA was precipitated with LiCl and reconstituted in nuclease-free water. Uncapped RNA was capped post-transcriptionally with Vaccinia Capping Enzyme (VCE) using the ScriptCap m7G Capping System (Epicentre Biotechnologies) as outlined in the user manual; replicons capped in this way are given the "v" prefix e.g. vA317 is the A317 replicon capped by VCE. Post-transcriptionally capped RNA was precipitated with LiCl and reconstituted in nuclease-free water. The concentration of the RNA samples was determined by measuring OD₂₆₀ₙₘ. Integrity of the *in vitro* transcripts was confirmed by denaturing agarose gel electrophoresis.

### Liposomal encapsulation

RNA was encapsulated in liposomes made by the method of references 9 and 40. The liposomes were made of 10% DSPC (zwitterionic), 40% DlinDMA (cationic), 48% cholesterol and 2% PEG-conjugated DMG (2kDa PEG). These proportions refer to the % moles in the total liposome.

DlinDMA (1,2-dilinoleyloxy-N,N-dimethyl-3-aminopropane) was synthesized using the procedure of reference 5. DSPC (1,2-Diastearoyl-sn-glycero-3-phosphocholine) was purchased from Genzyme. Cholesterol was obtained from Sigma-Aldrich. PEG-conjugated DMG (1,2-dimyristoyl-sn-glycero-3-phosphoethanolamine-N-[methoxy(polyethylene glycol), ammonium salt), DOTAP (1,2-dioleoyl-3-trimethylammonium-propane, chloride salt) and DC-chol (3β-[N-(N',N'-dimethylaminoethane)-carbamoyl]cholesterol hydrochloride) were from Avanti Polar Lipids.

In general, eight different methods have been used for preparing liposomes according to the invention. These are referred to in the text as methods (A) to (H) and they differ mainly in relation to filtration and TFF steps. Details are as follows:
(A) Fresh lipid stock solutions in ethanol were prepared. 37 mg of DlinDMA, 11.8 mg of DSPC, 27.8 mg of Cholesterol and 8.07 mg of PEG DMG 2000 were weighed and dissolved in 7.55 mL of ethanol. The freshly prepared lipid stock solution was gently rocked at 37°C for about 15 min to form a homogenous mixture. Then, 755 µL of the stock was added to 1.245 mL ethanol to make a working lipid stock solution of 2 mL. This amount of lipids was used to form liposomes with 250 µg RNA. A 2 mL working solution of RNA was also prepared from a stock solution of ~ 1µg/µL in 100 mM citrate buffer (pH 6). Three 20 mL glass vials (with stir bars) were rinsed with RNase Away solution (Molecular BioProducts, San Diego, CA) and washed with plenty of MilliQ water before use to decontaminate the vials of RNases. One of the vials was used for the RNA working solution and the others for collecting the lipid and RNA mixes (as described later). The working lipid and RNA solutions were heated at 37°C for 10 min before being loaded into 3cc luer-lok syringes. 2 mL of citrate buffer (pH 6) was loaded in another 3 cc syringe. Syringes containing RNA and the lipids were connected to a T mixer (PEEK^{™} 500 µm ID junction, Idex Health Science, Oak Harbor, WA) using FEP tubing (fluorinated ethylene-propylene; al FEP tubing has a 2mm internal diameter x 3mm outer diameter, supplied by Idex Health Science). The outlet from the T mixer was also FEP tubing. The third syringe containing the citrate buffer was connected to a separate piece of FEP tubing. All syringes were then driven at a flow rate of 7 mL/min using a syringe pump. The tube outlets were positioned to collect the mixtures in a 20 mL glass vial (while stirring). The stir bar was taken out and the ethanol/aqueous solution was allowed to equilibrate to room temperature for 1 hour. 4 ml of the mixture was loaded into a 5 cc syringe, which was connected to a piece of FEP tubing and in another 5 cc syringe connected to an equal length of FEP tubing, an equal amount of 100 mM citrate buffer (pH 6) was loaded. The two syringes were driven at 7mL/min flow rate using the syringe pump and the final mixture collected in a 20 mL glass vial (while stirring). Next, the mixture collected from the second mixing step (liposomes) were passed through a Mustang Q membrane (an anion-exchange support that binds and removes anionic molecules, obtained from Pall Corporation, AnnArbor, MI, USA). Before passing the liposomes, 4 mL of 1 M NaOH, 4 mL of 1 M NaCl and 10 mL of 100 mM citrate buffer (pH 6) were successively passed through the Mustang membrane. Liposomes were warmed for 10 min at 37°C before passing through the membrane. Next, liposomes were concentrated to 2 mL and dialyzed against 10-15 volumes of IX PBS using TFF before recovering the final product. The TFF system and hollow fiber filtration membranes were purchased from Spectrum Labs and were used according to the manufacturer's guidelines. Polysulfone hollow fiber filtration membranes (part number P/N: X1AB-100-20P) with a 100 kD pore size cutoff and 8 cm² surface area were used. For *in vitro* and *in vivo* experiments, formulations were diluted to the required RNA concentration with IX PBS.
(B) As method (A) except that, after rocking, 226.7 µL of the stock was added to 1.773 mL ethanol to make a working lipid stock solution of 2 mL, thus modifying the lipid:RNA ratio.
(C) As method (B) except that the Mustang filtration was omitted, so liposomes went from the 20 mL glass vial into the TFF dialysis.
(D) As method (C) except that the TFF used polyethersulfone (PES) hollow fiber membranes (part number P-C1-100E-100-01N) with a 100 kD pore size cutoff and 20 cm² surface area.
(E) As method (D) except that a Mustang membrane was used, as in method (A).
(F) As method (A) except that the Mustang filtration was omitted, so liposomes went from the 20 mL glass vial into the TFF dialysis.
(G) As method (D) except that a 4 mL working solution of RNA was prepared from a stock solution of ~ 1µg/µL in 100 mM citrate buffer (pH 6). Then four 20 mL glass vials were prepared in the same way. Two of them were used for the RNA working solution (2 mL in each vial) and the others for collecting the lipid and RNA mixes, as in (C). Rather than use T mixer, syringes containing RNA and the lipids were connected to a Mitos Droplet junction Chip (a glass microfluidic device obtained from Syrris, Part no. 3000158) using PTFE tubing (0.03 inches internal diameter x 1/16 inch outer diameter) using a 4-way edge connector (Syrris). Two RNA streams and one lipid stream were driven by syringe pumps and the mixing of the ethanol and aqueous phase was done at the X junction (100 µm x 105 µm) of the chip. The flow rate of all three streams was kept at 1.5 mL/min, hence the ratio of total aqueous to ethanolic flow rate was 2:1. The tube outlet was positioned to collect the mixtures in a 20 mL glass vial (while stirring). The stir bar was taken out and the ethanol/aqueous solution was allowed to equilibrate to room temperature for 1 h. Then the mixture was loaded in a 5 cc syringe, which was fitted to another piece of the PTFE tubing; in another 5 cc syringe with equal length of PTFE tubing, an equal volume of 100 mM citrate buffer (pH 6) was loaded. The two syringes were driven at 3mL/min flow rate using a syringe pump and the final mixture collected in a 20 mL glass vial (while stirring). Next, liposomes were concentrated to 2 mL and dialyzed against 10-15 volumes of IX PBS using TFF, as in (D).
(H) As method (A) except that the 2mL working lipid stock solution was made by mixing 120.9 µL of the lipid stock with 1.879 mL ethanol. Also, after mixing in the T mixer the liposomes from the 20mL vial were loaded into Pierce Slide-A-Lyzer Dialysis Cassette (Thermo Scientific, extra strength, 0.5-3 mL capacity) and dialyzed against 400-500 mL of IX PBS overnight at 4°C in an autoclaved plastic container before recovering the final product. Methods (A) to (H) as disclosed above use a N:P ratio of 8:1 for a specified amount of RNA. This ratio can readily be varied by changing the concentration of RNA in the RNA working solution.

### pKa measurement

The pKa of a lipid is measured in water at standard temperature and pressure using the following technique:
- 2mM solution of lipid in ethanol is prepared by weighing the lipid and dissolving in ethanol. 0.3mM solution of fluorescent probe toluene nitrosulphonic acid (TNS) in ethanol:methanol 9:1 is prepared by first making 3mM solution of TNS in methanol and then diluting to 0.3mM with ethanol.
- An aqueous buffer containing sodium phosphate, sodium citrate sodium acetate and sodium chloride, at the concentrations 20mM, 25mM, 20mM and 150 mM, respectively, is prepared. The buffer is split into eight parts and the pH adjusted either with 12N HCl or 6N NaOH to 4.44-4.52, 5.27, 6.15-6.21, 6.57, 7.10-7.20, 7.72-7.80, 8.27-8.33 and 10.47-11.12. 400µL of 2mM lipid solution and 800µL of 0.3mM TNS solution are mixed.
- 7.5µL of probe/lipid mix are added to 242.5µL of buffer in a 1mL 96 well plate. This is done with all eight buffers. After mixing, 100µL of each probe/lipid/buffer mixture is transferred to a 250µL black with clear bottom 96 well plate *(e.g.* model COSTAR 3904, Corning). A convenient way of performing this mixing is to use the Tecan Genesis RSP150 high throughput liquid handler and Gemini Software.
- Fluorescence of each probe/lipid/buffer mixture is measured (e.g. with a SpectraMax M5 spectrophotometer and SoftMax pro 5.2 software) with 322nm excitation, 431nm emission (auto cutoff at 420nm).
- After the measurement, the background fluorescence value of an empty well on the 96 well plate is subtracted from each probe/lipid/buffer mixture. The fluorescence intensity values are then normalized to the value at lowest pH. The normalized fluorescence intensity is then plotted against pH and a line of best fit is provided.
- The point on the line of best fit at which the normalized fluorescence intensity is equal to 0.5 is found. The pH corresponding to normalized fluorescence intensity equal to 0.5 is found and is considered the pKa of the lipid.

This method gives a pKa of 5.8 for DLinDMA, a preferred cationic lipid for use with the invention.

### Varying the N:P ratio for immunogen delivery

Self-replicating replicon (vA317) encoding RSV F protein. BALB/c mice, 4 or 8 animals per group, were given bilateral intramuscular vaccinations (50 µL per leg) on days 0 and 21 with the replicon (1µg) alone or formulated as liposomes with the RV01, RV05 or RV13. The RV01 liposomes had 40% DlinDMA, 10% DSPC, 48% cholesterol and 2% PEG-DMG. The RV05(01) liposomes had 40% cationic lipid, 48% cholesterol, 10% DSPC, and 2% PEG-DMG; the RV05(02) liposomes had 60% cationic lipid, 38% cholesterol, and 2% PEG-DMG. The RV13 liposomes had 40% DOTAP, 10% DPE, 48% cholesterol and 2% PEG-DMG. For comparison, naked plasmid DNA (20 µg) expressing the same RSV-F antigen was delivered either using electroporation or with RV01(10) liposomes (0.1µg DNA). Four mice were used as a naïve control group.

These liposomes were prepared by method (D), except for RV01(05) which used method (B). The RNA concentration was varied to give different N:P ratios as shown in the table below.

The Z average particle diameter, polydispersity index and encapsulation efficiency of the liposomes were as follows, also showing the N:P ratio:

| **RV** | **Zav (nm)** | **pdI** | **% encapsulation** | **N:P ratio** |
|---|---|---|---|---|
| RV01 (10) | 158.6 | 0.088 | 90.7 | 8:1 |
| RV01 (08) | 156.8 | 0.144 | 88.6 | 16:1 |
| RV01 (05) | 136.5 | 0.136 | 99 | 8:1 |
| RV01 (09) | 153.2 | 0.067 | 76.7 | 4:1 |
| RV05 (01) | 148 | 0.127 | 80.6 | 8:1 |
| RV05 (02) | 177.2 | 0.136 | 72.4 | 8:1 |
| RV01 (10) | 134.7 | 0.147 | 87.8 ^{∗} | 8:1 |
| RV13 (02) | 128.3 | 0.179 | 97 | 8:1 |

| | | | | |
|---|---|---|---|---|
| ^{∗} For this RV01(10) formulation the nucleic acid was DNA not RNA | | | | |

Serum was collected for antibody analysis on days 14, 36 and 49. Spleens were harvested from mice at day 49 for T cell analysis.

F-specific serum IgG titers (GMT) were as follows:

| **RV** | **Day 14** | **Day 36** |
|---|---|---|
| Naked DNA plasmid | 439 | 6712 |
| Naked A317 RNA | 78 | 2291 |
| RV01 (10) | 3020 | 26170 |
| RV01 (08) | 2326 | 9720 |
| RV01 (05) | 5352 | 54907 |
| RV01 (09) | 4428 | 51316 |
| RV05 (01) | 1356 | 5346 |
| RV05 (02) | 961 | 6915 |
| RV01 (10) DNA | 5 | 13 |
| RV13 (02) | 644 | 3616 |

The proportion of T cells which are cytokine-positive and specific for RSV F51-66 peptide are as follows, showing only figures which are statistically significantly above zero:

| **RV** | **CD4+CD8-** | | | | **CD4-CD8+** | | | |
|---|---|---|---|---|---|---|---|---|
| | IFNγ | IL2 | IL5 | TNFα | IFNγ | IL2 | IL5 | TNFα |
| Naked DNA plasmid | 0.04 | 0.07 | | 0.10 | 0.57 | 0.29 | | 0.66 |
| Naked A317 RNA | 0.04 | 0.05 | | 0.08 | 0.57 | 0.23 | | 0.67 |
| RV01 (10) | 0.07 | 0.10 | | 0.13 | 1.30 | 0.59 | | 1.32 |
| RV01 (08) | 0.02 | 0.04 | | 0.06 | 0.46 | 0.30 | | 0.51 |
| RV01 (05) | 0.08 | 0.12 | | 0.15 | 1.90 | 0.68 | | 1.94 |
| RV01 (09) | 0.06 | 0.08 | | 0.09 | 1.62 | 0.67 | | 1.71 |
| RV05 (01) | | | | | 0.06 | 0.04 | | 0.19 |
| RV05 (02) | 0.05 | 0.07 | | 0.11 | 0.64 | 0.35 | | 0.69 |
| RV01 (10) DNA | | | | 0.03 | | | | 0.08 |
| RV13 (02) | 0.03 | 0.04 | | 0.06 | 1.15 | 0.41 | | 1.18 |

Thus the liposome formulations significantly enhanced immunogenicity relative to the naked RNA controls, as determined by increased F-specific IgG titers and T cell frequencies. Plasmid DNA formulated with liposomes, or delivered naked using electroporation, was significantly less immunogenic than liposome-formulated self-replicating RNA.

The RV01 and RV05 RNA vaccines were more immunogenic than the RV13 (DOTAP) vaccine. These formulations had comparable physical characteristics and were formulated with the same self-replicating RNA, but they contain different cationic lipids. RV01 and RV05 both have a tertiary amine in the headgroup with a pKa of about 5.8, and also include unsaturated alkyl tails. RV13 has unsaturated alkyl tails but its headgroup has a quaternary amine and is very strongly cationic. These results suggest that lipids with tertiary amines with pKas in the range 5.0 to 7.6 are superior to lipids such as DOTAP, which are strongly cationic, when used in a liposome delivery system for RNA.

The N:P ratio had an impact on immunogenicity, with 4:1 (RV01(09)) > 8:1 (RV01(10)) > 16:1 (RV01(08)).

It will be understood that the invention has been described by way of example only and modifications may be made whilst remaining within the scope and spirit of the invention.

**Table 1: useful phospholipids**

| | |
|---|---|
| DDPC | 1,2-Didecanoyl-sn-Glycero-3-phosphatidylcholine |
| DEPA | 1,2- Dierucoyl-sn-G lycero- 3 -Phosphate |
| DEPC | 1,2-Erucoyl-sn-Glycero-3-phosphatidylcholine |
| DEPE | 1,2-Dierucoyl-sn-Glycero-3-phosphatidylethanolamine |
| DEPG | 1,2-Dierucoyl-sn-Glycero-3[Phosphatidyl-rac-(1-glycerol...) |
| DLOPC | 1,2- Linoleoyl-sn -G lycero- 3 -phosphatidylcholine |
| DLPA | 1,2-Dilauroyl-sn-Glycero-3-Phosphate |
| DLPC | 1,2-Dilauroyl-sn-Glycero-3-phosphatidylcholine |
| DLPE | 1,2-Dilauroyl-sn-Glycero-3-phosphatidylethanolamine |
| DLPG | 1,2-Dilauroyl-sn-Glycero-3[Phosphatidyl-rac-(1-glycerol...) |
| DLPS | 1,2- Dilauroyl-sn-Glycero- 3 -phosphatidylserine |
| DMG | 1,2-Dimyristoyl-sn-glycero-3-phosphoethanolamine |
| DMPA | 1,2-Dimyristoyl-sn-Glycero-3-Phosphate |
| DMPC | 1,2-Dimyristoyl-sn -G lycero- 3 -phosphatidy lcholine |
| DMPE | 1,2-Dimyristoyl-sn-Glycero-3-phosphatidylethanolamine |
| DMPG | 1,2- Myristoyl-sn-Glycero-3[Phosphatidyl-rac-(1-glycerol...) |
| DMPS | 1,2-Dimyristoyl-sn-Glycero-3-phosphatidylserine |
| DOPA | 1,2-Dioleoyl-sn-Glycero-3-Phosphate |
| DOPC | 1,2-Dioleoyl-sn-Glycero-3-phosphatidylcholine |
| DOPE | 1,2-Dioleoyl-sn-Glycero-3-phosphatidylethanolamine |
| DOPG | 1,2-Dioleoyl-sn-Glycero-3[Phosphatidyl-rac-(1-glycerol...) |
| DOPS | 1,2-Dioleoyl-sn-Glycero-3-phosphatidylserine |
| DPPA | 1,2-Dipalmitoyl-sn-Glycero-3-Phosphate |
| DPPC | 1,2- Dipalmitoyl-sn -Glycero- 3 -phosphatidy lcholine |
| DPPE | 1,2-Dipalmitoyl-sn-Glycero-3-phosphatidylethanolamine |
| DPPG | 1,2-Dipalmitoyl-sn-Glycero-3[Phosphatidyl-rac-(1-glycerol...) |
| DPPS | 1,2-Dipalmitoyl-sn-Glycero-3-phosphatidylserine |
| DPyPE | 1,2-diphytanoyl-sn-glycero-3-phosphoethanolamine |
| DSPA | 1,2- Distearoyl-sn-Glycero- 3-Phosphate |
| DSPC | 1,2-Distearoyl-sn-Glycero-3-phosphatidylcholine |
| DSPE | 1,2-Diostearpyl-sn-Glycero-3-phosphatidylethanolamine |
| DSPG | 1,2-Distearoyl-sn-Glycero-3[Phosphatidyl-rac-(1-glycerol...) |
| DSPS | 1,2-Distearoyl-sn-Glycero-3-phosphatidylserine |
| EPC | Egg-PC |
| HEPC | Hydrogenated Egg PC |
| HSPC | High purity Hydrogenated Soy PC |
| HSPC | Hydrogenated Soy PC |
| LYSOPC | MYRISTIC 1-Myristoyl-sn-Glycero-3-phosphatidylcholine |
| LYSOPC PALMITIC | 1-Pabnitoyl-sn-Glycero-3-phosphatidylcholine |
| LYSOPC STEARIC | 1-Stearoyl-sn-Glycero-3-phosphatidylcholine |
| Milk Sphingomyelin MPPC | 1-Myristoyl,2-palmitoyl-sn-Glycero 3-phosphatidylcholine |
| MSPC | 1-Myristoyl,2-stearoyl-sn-Glycero-3-phosphatidylcholine |
| PMPC | 1-Palmitoyl,2-myristoyl-sn-Glycero-3-phosphatidylcholine |
| POPC | 1-Palmitoyl,2-oleoyl-sn-Glycero-3-phosphatidylcholine |
| POPE | 1-Palmitoyl-2-oleoyl-sn-Glycero-3-phosphatidylethanolamine |
| POPG | 1,2-Dioleoyl-sn-Glycero-3[Phosphatidyl-rac-(1-glycerol)...] |
| PSPC | 1-Palmitoyl,2-stearoyl-sn-Glycero-3-phosphatidylcholine |
| SMPC | 1-Stearoyl,2-myristoyl-sn-Glycero-3-phosphatidylcholine |
| SOPC | 1-Stearoyl,2-oleoyl-sn-Glycero-3-phosphatidylcholine |
| SPPC | 1-Stearoyl,2-palmitoyl-sn-Glycero-3-phosphatidylcholine |

### REFERENCES

[1] WO2005/121348.
[2] WO2008/137758.
[3] WO2009/086558.
[4] WO2011/076807.
[5] Heyes et al. (2005) J Controlled Release 107:276-87.
*[6]* Liposomes: Methods and Protocols, Volume 1: Pharmaceutical Nanocarriers: Methods and Protocols. (ed. Weissig). Humana Press, 2009. ISBN 160327359X.
*[7]* Liposome Technology, volumes I, II & III. (ed. Gregoriadis). Informa Healthcare, 2006.
*[8]* Functional Polymer Colloids and Microparticles volume 4 (Microspheres, microcapsules & liposomes). (eds. Arshady & Guyot). Citus Books, 2002.
[9] Jeffs et al. (2005) Pharmaceutical Research 22 (3):362-372.
[10] Martinon et al. (1993) Eur J Immunol 23:1719-22.
[11] WO2005/113782.
[12] WO2011/005799.
[13] Giuliani et al. (2006) Proc Natl Acad Sci U S A 103(29):10834-9.
[14] WO2009/016515.
[15] WO02/34771.
[16] WO2005/032582.
[17] WO2010/119343.
[18] WO2006/110413.
[19] WO2005/111066.
[20] WO2005/002619.
[21] WO2006/138004.
[22] WO2009/109860.
[23] WO02/02606.
[24] WO03/018054.
[25] WO2006/091517.
[26] WO2008/020330.
[27] WO2006/089264.
[28] WO2009/104092.
[29] WO2009/031043.
[30] WO2007/049155.
[31] Gennaro (2000) Remington: The Science and Practice of Pharmacy. 20th edition, ISBN: 0683306472.
*[32]* Methods In Enzymology (S. Colowick and N. Kaplan, eds., Academic Press, Inc.)
[33] Handbook of Experimental Immunology, Vols. I-IV (D.M. Weir and C.C. Blackwell, eds, 1986, Blackwell Scientific Publications)
[34] Sambrook et al. (2001) Molecular Cloning: A Laboratory Manual, 3rd edition (Cold Spring Harbor Laboratory Press).
[35] Handbook of Surface and Colloidal Chemistry (Birdi, K.S. ed., CRC Press, 1997)
[36] Ausubel et al. (eds) (2002) Short protocols in molecular biology, 5th edition (Current Protocols).
*[37]* Molecular Biology Techniques: An Intensive Laboratory Course, (Ream et al., eds., 1998, Academic Press)
[38] PCR (Introduction to Biotechniques Series), 2nd ed. (Newton & Graham eds., 1997, Springer Verlag)
[39] Yoneyama & Fujita (2007) Cytokine & Growth Factor Reviews 18:545-51.
[40] Maurer et al. (2001) Biophysical Journal, 80: 2310-2326.

### EMBODIMENTS

1. A liposome in which an immunogen-encoding RNA is encapsulated, wherein the liposome comprises a cationic lipid and the liposome & RNA have a N:P ratio of between 1:1 and 20:1.
2. The liposome of embodiment 1, having a diameter in the range of 50-220nm.
3. The liposome of any preceding embodiment, wherein the RNA is a self-replicating RNA
4. The liposome of embodiment 3, wherein the self-replicating RNA encodes (i) a RNA-dependent RNA polymerase which can transcribe RNA from the self-replicating RNA molecule and (ii) an immunogen.
5. The liposome of embodiment 4, wherein the RNA has two open reading frames, the first of which encodes an alphavirus replicase and the second of which encodes the immunogen.
6. The liposome of any preceding embodiment, wherein the RNA is 9000-12000 nucleotides long.
7. The liposome of any preceding embodiment, wherein the immunogen can elicit an immune response *in vivo* against a bacterium, a virus, a fungus or a parasite.
8. The liposome of embodiment 7, wherein the immunogen can elicit an immune response *in vivo* against respiratory syncytial virus glycoprotein F.
9. The liposome of any preceding embodiment, wherein the liposome & RNA have a N:P ratio between 2:1 and 18:1.
10. The liposome of any preceding embodiment, wherein the liposome & RNA have a N:P ratio between 3:1 and 11:1.
11. The liposome of any preceding embodiment, wherein the liposome & RNA have a N:P ratio between 4:1 and 16:1.
12. The liposome of any preceding embodiment, wherein the liposome & RNA have a N:P ratio between 6:1 and 14:1.
13. The liposome of any preceding embodiment, wherein the liposome & RNA have a N:P ratio between 8:1 and 12:1.
14. The liposome of any one of embodiments 1 to 9, wherein the liposome & RNA have a N:P ratio of 2:1,4:1,8:1 or 10:1.
15. A pharmaceutical composition comprising a liposome of any preceding embodiment.
16. A method for raising a protective immune response in a vertebrate, comprising the step of administering to the vertebrate an effective amount of the liposome of embodiments 1-14, or the pharmaceutical composition of embodiment 15.

## Claims

1. A liposome in which an immunogen-encoding RNA is encapsulated, wherein the liposome comprises a cationic lipid and the liposome & RNA have a N:P ratio of between 1:1 and 20:1.

2. The liposome of claim 1, having a diameter in the range of 50-220nm.

3. The liposome of any preceding claim, wherein the RNA is a self-replicating RNA, optionally wherein the self-replicating RNA encodes (i) a RNA-dependent RNA polymerase which can transcribe RNA from the self-replicating RNA molecule and (ii) an immunogen, further optionally wherein the RNA has two open reading frames, the first of which encodes an alphavirus replicase and the second of which encodes the immunogen.

4. The liposome of any preceding claim, wherein the RNA is 9000-12000 nucleotides long.

5. The liposome of any preceding claim, wherein the immunogen can elicit an immune response *in vivo* against a virus, a bacterium, a fungus, a parasite, a tumour or an allergen, optionally wherein the immunogen can elicit an immune response *in vivo* against respiratory syncytial virus glycoprotein F.

6. The liposome of claim 5, wherein the immunogen is a surface polypeptide, optionally wherein the immunogen is a spike glycoprotein, a hemagglutinin, an envelope glycoprotein or an adhesin.

7. The liposome of any preceding claim, wherein the liposome & RNA have a N:P ratio of between 2:1 and 16:1, optionally between 3:1 and 11:1.

8. The liposome of claim 7, wherein the liposome & RNA have a N:P ratio between 4:1 and 7:1.

9. The liposome of claim 8, wherein the liposome & RNA have a N:P ratio between 4:1 and 6:1.

10. A pharmaceutical composition comprising a liposome of any preceding claim.

11. The pharmaceutical composition of claim 10, for use in a method of raising a protective immune response in a vertebrate.

12. The pharmaceutical composition for use of claim 11, for use as a prophylactic vaccine.

13. The pharmaceutical composition for use of claim 12 or 13, administered by the intramuscular or intradermal route.

14. A process for preparing a liposome which encapsulates an immunogen-encoding RNA, wherein the liposome is formed by mixing liposome-forming components with an immunogen-encoding RNA, wherein the liposome-forming components comprise a cationic lipid, and wherein the cationic lipid and the RNA are mixed at a N:P ratio of between 1:1 and 20:1.

15. The process of claim 14, further comprising formulation into a pharmaceutical composition comprising a pharmaceutically acceptable carrier, in addition to the liposome.
